(19) Europäisches Patentamt
European Patent Office
Office européen des brevets

(11) Veröffentlichungsnummer: **0 001 584**
**B1**

# (12) EUROPÄISCHE PATENTSCHRIFT

(45) Veröffentlichungstag der Patentschrift: **30.06.82**

(21) Anmeldenummer: **78101050.9**

(22) Anmeldetag: **02.10.78**

(51) Int. Cl.³: **C 07 F 9/38,** C 07 F 9/65, A 61 K 7/16, A 61 K 31/66, A 61 K 49/02, C 02 F 5/14, C 11 D 3/36

## (54) Hydroxydiphosphonsäuren, Verfahren zu ihrer Herstellung und ihre Verwendung als Komplexierungsmittel.

(30) Priorität: **07.10.77 DE 2745083**

(43) Veröffentlichungstag der Anmeldung:
**02.05.79 Patentblatt 79/9**

(45) Bekanntmachung des Hinweises auf die Patenterteilung:
**30.06.82 Patentblatt 82/26**

(84) Benannte Vertragsstaaten:
**BE CH FR GB NL**

(56) Entgegenhaltungen:
**DE - A - 2 343 196**
**DE - A - 2 534 391**
**DE - B - 2 130 794**
**FR - A - 2 354 338**

(73) Patentinhaber: **Henkel Kommanditgesellschaft auf Aktien**
**Postfach 1100 Henkelstrasse 67**
**D-4000 Düsseldorf 1 (DE)**

(72) Erfinder: **Blum, Helmut**
**Am Falder 20**
**D-4000 Düsseldorf 13 (DE)**
Erfinder: **Hempel, Hans-Ulrich, Dr.**
**Wiesengrund 5**
**D-5063 Overath (DE)**
Erfinder: **Worms, Karl-Heinz, Dr.**
**Am Nettchesfeld 10**
**D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 584 B1

# Hydroxydiphosphonsäuren, Verfahren zu ihrer Herstellung und ihre Verwendung als Komplexierungsmittel

Gegenstand der Erfindung sind neue 1-Hydroxy-1,1-diphosphonsäuren und deren wasserlösliche Salze. Die neuen Verbindungen sind gute Komplexbildner und weisen wertvolle anwendungstechnische Eigenschaften auf.

Aminoalkandiphosphonsäuren, die ggf. noch eine Hydroxygruppe enthalten, gewinnen in letzter Zeit zunehmendes Interesse wegen ihrer guten Wirksamkeit als Komplexbildner, insbesondere auch in unterstöchiometrischen Mengen (Threshold-Substanzen) sowie in pharmazeutischen Produkten.

Verbindungen dieser Art sind beispielsweise 3-Aminoalkan-1,1-diphosphonsäuren wie beispielsweise 1,3-Diamino-propan-1,1-diphosphonsäure oder 3-Amino-1-hydroxypropan-1,1-diphosphonsäure. Trotz ihrer guten Wirksamkeit können diese bekannten Verbindungen nicht in allen Eigenschaften befriedigen. So besitzen sie nur einen mäßigen Threshold-Effekt bei niedriger Dosierung. Außerdem treten in bestimmten pH-Bereichen teilweise relativ schwerlösliche Erdalkalikomplexe auf.

Es wurde überraschenderweise gefunden, daß die neuen nachstehend beschriebenen Hydroxydiphosphonsäuren und deren wasserlösliche Salze besonders gute Eigenschaften besitzen.

Die neuen 1-Hydroxy-1,1-diphosphonsäuren entsprechen der allgemeinen Formel

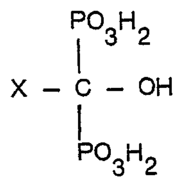

(I)

wobei

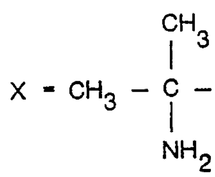

oder

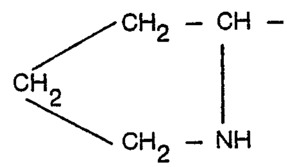

bedeutet.

Verbindungen der Formel 1 können hergestellt werden, indem man 2-Aminoisobuttersäure oder Prolin mit phosphoriger Säure und Phosphortrichlorid, wobei das Molverhältnis 1:1:1 bis 1:3:3 beträgt, umsetzt und das Reaktionsprodukt sauer hydrolysiert. Phosphorige Säure und Phosphortrichlorid brauchen im übrigen nicht in dem gleichen Molverhältnis vorzuliegen. Die Umsetzung erfolgt bei einer Temperatur von 50°C bis 140°C, vorzugsweise in Temperaturbereich von 70°C bis 120°C.

Bei der Umsetzung von 2-Aminoisobuttersäure mit phosphoriger Säure und Phosphortrichlorid hat es sich als besonders geeignet erwiesen, die Umsetzung der genannten Stoffe im Molverhältnis 1:1,5:1,5 durchzuführen. Verwendet man als Ausgangssubstanz dagegen Prolin (2-Pyrrolidincarbonsäure), so ist es vorteilhaft, das Molverhältnis zu phosphoriger Säure und zu Phosphortrichlorid im Bereich von 1:2:2 zu wählen.

Es ist weiterhin zweckmäßig—wenn auch nicht notwendig-, die Umsetzung in Gegenwart von inerten Lösungsmitteln wie Chlorbenzol, Tetrachloräthan oder Dioxan durchzuführen. Eine praktische Arbeitsweise besteht darin, zunächst das Lösungsmittel mit der Aminocarbonsäure und der phosphorigen Säure zu vermischen und das Gemisch auf eine Temperatur von vorzugsweise 70°C bis 120°C aufzuheizen, um anschließend langsam Phosphortrichlorid hinzuzufügen. Der Ansatz wird dann noch eine Zeitlang auf dieser erhöhten Temperatur belassen, wonach die saure Hydrolyse erfolgt.

Dabei ist es jedoch nicht erforderlich, eine Säure hinzuzufügen, sondern es genügt, dem sauren Gemisch eine entsprechende Menge Wasser hinzuzufügen. Das Hydrolysat wird nach bekannten Methoden aufgearbeitet, indem beispielsweise nach einer Filtration die wäßrige Phase abgetrennt und ggf. nach Einengung die Phosphonsäure durch Zusatz von geeigneten Lösungsmitteln wie Aceton oder Aceton-Alkohol-Gemischen ausgefällt wird.

Die neuen Hydroxydiphosphonsäuren können durch vollständige oder teilweise Neutralisation mit anorganischen, organischen oder quartären Basen wie NaOH, KOH, $NH_4OH$, Alkalicarbonaten, Alkanolaminen wie Monoäthanolamin, Diäthanolamin und Triäthanolamin oder Tetraalkylammoniumhydroxid in die entsprechenden wasserlöslichen Salze überführt werden.

Die neuen Hydroxydiphosphonsäuren einschließlich ihrer Alkali-, Ammonium- oder Alkanolaminsalze sind gute Komplexbildner für Erdalkali-, vorzugsweise Calciumionen und können daher speziell für Vorgänge der Wasserenthärtung Verwendung finden.

Es ist daher nicht notwendig, mit stöchiometrischen Mengen zu arbeiten, sondern man kann auch

durch Anwendung unterstöchiometrischer Mengen- gegebenenfalls sogar mit Mengen von 0,2 bis 5 mg/l-Calcitfällungen erheblich verzögern.

Sie sind daher auch als Korrosions- und Steinansatzverhütungsmittel für Kühlwasser, insbesondere in Kombination mit an sich bekannten Zusätzen wie beispielsweise zweiwertige Zink- und/oder Cadmiumsalze, Orthophosphate, Chromate oder Hydrazinhydrat gut geeignet.

Was je nach der zur Anwendung gelangenden Verbindung als stöchiometrische Menge anzusehen ist, läßt sich durch einen einfachen Versuch leicht ermitteln. Im allgemeinen werden die Komplexbildner in Mengen von 1 Mol per 2000 Mol Metallionen bis zur sechsfachen stöchiometrischen Menge verwendet.

Die genannten Eigenschaften bewirken, daß die neuen Komplexbildner beispielsweise auch für die Entkrustung von Geweben, in denen Alkalisalze sich abgelagert haben, und die Verminderung der Ascheanreicherung in Geweben Anwendung finden können. Sie sind weiterhin geeignet für Reinigungsprozesse von starren Gegenständen wie insbesondere Metall oder Glas. Hierbei kommt insbesondere die Verwendung als Zusatz zu Flaschenspülmitteln in Betracht.

Von dem Komplexbildungsvermögen kann man in vorteilhafter Weise auch Gebrauch machen in Systemen, in denen Kupferionen einen unerwünschten Einfluß haben. Als Beispiele sind hier die Vermeidung der Zersetzung von Perverbindungen oder auch die Stabilisierung von Fetten und Seifen zu nennen. Weiterhin sind die genannten Verbindungen als Zusatz zu Färbebädern von Textilien geeignet, um Metallionen, die unerwünschte Farbnuancen bilden, komplex zu binden.

Schließlich kann das Vermögen der Komplexbildung auch dazu verwendet werden, um Pflanzen sogenannte Spurenelemente zuzuführen. Das gute Komplexbildungsvermögen dieser Verbindungen zeigt sich auch daran, daß die bekannte Rotfärbung nicht eintritt, welche sonst bei Zusatz von Rhodanid zu Lösungen, die dreiwertiges Eisen enthalten, beobachtet wird. Man kann daher diese Eigenschaften auch in vorteilhafter Weise dazu verwenden, um das Absetzen von Eisenverbindungen, insbesondere Eisenhydroxid auf Geweben oder beim Flaschenspülen zu verhindern. Ebenfalls können die nauen Verbindungen anstelle von Cyaniden in galvanischen Bädern eingesetzt werden.

Schließlich kommen sie auch als Buildersubstanzen mit komplexierenden Eigenschaften in Wasch- und Reinigungsmitteln in Frage und können in Kombination mit bekannten anionenaktiven, kationenaktiven oder nichtionogenen Netzmitteln verwendet werden. Weiterhin können sie in Kombination Atzalkalien, Alkalicarbonaten, -silikaten, -phosphaten oder -boraten Anwendung finden.

Die beschriebenen Phosphonsäuren sind auch als Wirkstoffe in pharmazeutischen oder kosmetischen Präparaten geeignet, die Anwendung finden, um Störungen des Calcium- bzw. Phosphatstoffwechsels sowie die damit verbundenen Erkrankungen therapeutisch oder prophylaktisch zu behandeln.

Zur pharmazeutischen Anwendung kommen anstelle der freien Säure auch ihre pharmakologisch unbedenklichen Salze wie Natrium-, Kalium-, Magnesium-, Ammonium- und substituierten Ammoniumsalze wie Mono-, Di- oder Triäthanolammoniumsalze in Frage. Sowohl die partiellen Salze, in denen nur ein Teil der aciden Protonen durch andere Kationen ersetzt ist, als auch Vollsalze können benutzt werden, jedoch sind partielle Salze, die in wäßriger Lösung annähernd neutral reagieren (pH 5—9), bevorzugt. Mischungen der vorgenannten Salze können ebenfalls angewandt werden.

Die Dosierung der verwendeten Verbindungen ist variabel und hängt von den jeweiligen Konditionen wie Art und Schwere der Erkrankung, Dauer der Behandlung und der jeweiligen Verbindung ab. Einzelne Dosierungen können von 0,05 bis 500 mg pro kg Körpergewicht betragen. Die bevorzugte Dosierung beträgt 1 bis 50 mg pro kg Körpergewicht und Tag und kann in bis zu 4 Portionen täglich verabreicht werden. Die höheren Dosierungen sind bei oraler Applikation infolge der begrenzten Resorption erforderlich.

Bei länger dauernden Behandlungen sind nach anfänglich höheren Dosierungen normalerweise geringere Dosierungen notwendig, um den gewünschten Effekt aufrechtzuerhalten.

Dosierungen unter 0,05 mg/kg Körpergewicht beeinflussen die pathologische Verkalkung bzw. die Auflösung von harten Geweben nur unerheblich. Bei Dosierungen von über 500 mg/kg Körpergewicht können langfristig toxische Nebenwirkungen auftreten. Die neuen Diphosphonsäuren bzw. ihre Salze können sowohl oral als auch in hypertonischer Lösung subkutan, intramuskulär oder intravenös appliziert werden. Die bevorzugten Dosisbereiche für diese Anwendungen sind (in mg/kg . Tag)

| | |
|---|---|
| oral | 1 —50 |
| subkutan | 1 —10 |
| intramuskulär | 0,05—10 |
| intravenös | 0,05— 2 |

Die Substanzen können zur Verabreichung in Tabletten, Pillen, Kapseln oder Injektionslösungen formuliert werden. Die Anwendung kann in Kombination mit dem Hormon Calcitonin erfolgen. Für Tiere können die Substanzen auch in Futter bzw. als Futterzusätze Verwendung finden.

Bei Anwendung in kosmetischen Präparaten wie Mund-und Zahnpflegemitteln verhindern die Hydroxydiphosphonsäuren bzw. ihre pharmakologisch unbedenklichen Salze in Konzentrationen von 0,01 bis 5% die Bildung von Zahnstein.

Schließlich sind die neuen Hydroxydiphosphonsäuren auch geeignet als Zusatz zu Präparaten für die Herstellung von $99^m$-Technetium-Radiodiagnostika. Durch Radiographie lassen sich nämlich Knochen- und Gewebeerkrankungen erkennen und lokalisieren. Zu diesem Zweck hat man in letzter Zeit das Isotop Technetium-$99^m$, welches eine Halbwertzeit von 6 Stunden aufweist, verwendet.

Zu seiner Herstellung stehen gut handbare Vorrichtungen zur Verfügung, aus denen durch Eluierung mit isotonischer Kochsalzlösung das radioaktive Isotop in Form von $99^m$-Pertechnetat erhalten werden kann.

Das Pertechnetat-$99^m$ unterscheidet sich von dem früher verwendeten radioaktiven Fluor oder Strontium darin, daß es im Körper nicht spezifisch im Skelett oder in kalkhaltigen Tomoren gebunden wird. Zu seiner Anwendung muß es daher zu einer niedrigen Oxidationsstufe reduziert werden und dann mit einem geeigneten Komplexbildner in dieser Oxidationsstufe stabilisiert werden. Der Komplexbildner muß weiterhin eine hohe Selektivität zur bevorzugten Absorption am Skelett bzw. an kalkhaltigen Tumoren aufweisen.

Es hat sich herausgestellt, daß sich für diese Zwecke die oben beschriebenen komplexierenden Hydroxydiphosphonsäuren bzw. deren pharmazeutisch unbedenklichen wasserlöslichen Salze besonders gut eignen. Dabei werden die Phosphonsäuren zusammen mit einem pharmazeutisch brauchbaren Zinn(II)-, Chrom(II)- oder Eisen(II)-Salz angewandt, wobei die reduzierenden Salze in stöchiometrischen untergeordneten Mengen, bezogen auf die Phosphonsäure oder deren wasserlösliche Salze, vorhanden sind. Dadurch wird die einfache Herstellung eines hochstabilen Produktes ermöglicht, welches zum Verkauf in fester Form als Tablette oder in Form einer Lösung, enthalten in einer Ampulle geeignet ist.

Die Tablette oder der Inhalt einer Ampulle bilden nach Zugabe zu einer Pertechnetat-Lösung ein sehr wirksames Mittel zur Diagnostik von Knochentumoren, lokalen Störungen des Knochenstoffwechsels sowie kalkeinlagernden Gewebetumoren.

Beispiel 1

*1-Hydroxy-2-amino-2-methylpropan-1,1-diphosphonsäure*

0,48 Mol 2-Aminoisobuttersäure wurden mit 0,72 Mol $H_3PO_3$ in 250 ml Chlorbenzol auf 100°C aufgeheizt und langsam 0,72 Mol $PCl_3$ zugetropft. Nach dreistündigem Nacherhitzen wurde das Reaktionsgemisch mit einem Überschuß an Wasser (240 ml) hydrolysiert. Die wäßrige Phase wurde vom Chlorbenzol getrennt und über Aktivkohle filtriert. Das wasserklare Hydrolysat wurde auf 180 ml eingedampft, mit der 8-fachen Volumenmenge Aceton gefällt und getrocknet.

Analysen:

| | | | | | | | |
|---|---|---|---|---|---|---|---|
| C | 19,32 | H | 5,03 | P | 24,90 | N | 5,62 |
| | (19,28) | | (5,22) | | (24,90) | | (5,63) |

Molgewicht: 249,9 (249,0); m.p. 217°C

Durch $^1H$- und $^{31}P$—NMR-Spektroskopie wurde die Struktur

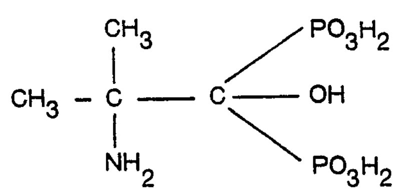

bestätigt.

Beispiel 2

Zu einem Gemisch von 1 Mol 2-Aminoisobuttersäure, 600 ml Chlorbenzol und 4 Mol $PCl_3$ wurden 6 Mol Wasser bei Zimmer-temperatur hinzugefügt. Das Reaktionsgemisch wurde anschließend 5 Stunden auf 100°C erhitzt. Anschließend wurde das Reaktionsprodukt mit 500 ml $H_2O$ hydrolysiert. Das Lösungsmittel wurde abgetrennt und der Ansatz analog dem Beispiel 1 aufgearbeitet.

Beispiel 3

*2'-Pyrrolidin-1-hydroxymethan-1,1-diphosphonsäure*

0,43 Mol Prolin und 0,86 Mol $H_3PO_3$ in 250 ml Dioxan wurden auf 80°C erhitzt und mit 0,86 Mol $PCl_3$ versetzt. Das Reaktionsgemisch wurde 20 Std. bei dieser Temperatur belassen, danach mit 500 ml Wasser hydrolysiert und über Aktivkohle filtriert. Die beim Einengen des Filtrats ausgefallene Diphosphonsäure wurde getrennt und bei 60°C im Vakuum getrocknet. Die Verbindung lag als Monohydrat vor und war chromatographisch rein.

Analysen:

| | | | |
|---|---|---|---|
| C 21,75 | H 5,19 | P 22,17 | N 5,02 |
| (21,50) | (5,42) | (22,20) | (5,02) |

Molgewicht: 278 (279,1); m.p. 195°C (Zers.)

Durch $^1$H- und $^{31}$P—NMR-Messungen wurde die Struktur bestätigt

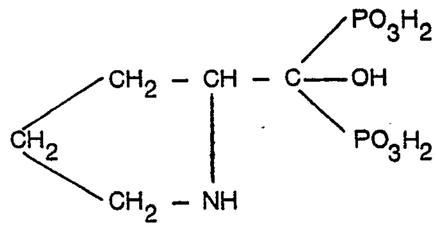

Beispiel 4

In der folgenden Tabelle ist die Treshold-Aktivatät der neuen Verbindungen im Vergleich mit bereits bekannten 3-Aminoalkan-1,1-diphosphonsäuren aufgeführt. Bestimmt wurde der härtestabilisierende Effekt wie folgt:

Lösungen mit einer Wasserhärte von 20°d.H. (4/5 Ca- und 1/5 Mg-Härte) und einem zusätzlichen Gehalt von 4,5 g $Na_2CO_3$/1, 0,6 g Natriumsilikat ($SiO_2$:$Na_2O$-Verhältnis=3,36) und 150 mg/1 des unten angegebenen Inhibitors wurden 30 Min. auf 95°C erhitzt. Anschließend wurde der in der Lösung verbliebene Anteil an Calcium flammenphotometrisch ermittelt.

| Nr. | Substanz | % CaO in Lösung |
|---|---|---|
| (1) | 1-Hydroxy-2-amino-2-methylpropan-1,1-diphosphonsäure | 94,3 |
| (2) | 2'-Pyrrolidin-1-hydroxymethan-1,1-diphosphonsäure | 75,8 |
| | 1,3-Diamino-propan-1,1-diphosphonsäure | 50,2 |
| | 3-Amino-1-hydroxypropan-1,1-diphosphonsäure | 35,7 |
| | 3-Amino-1-hydroxybutan-1,1-diphosphonsäure (bekannt aus DE.A.2534391) | 68,2 |

**Patentansprüche**

1. Hydroxydiphosphonsäuren der allgemeinen Formel

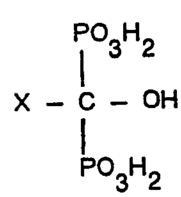

(I)

wobei

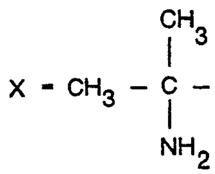

oder

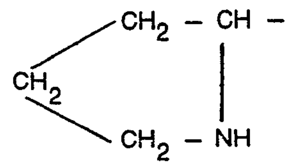

bedeutet und deren wasserlösliche Salze.

2. Verfahren zur Herstellung von Hydroxydiphosphonsäuren der Formel I, dadurch gekennzeichnet, daß man 2-Aminoisobuttersäure bzw. Prolin mit phosphoriger Säure und Phosphortrichlorid, wobei das Molverhältnis 1:1:1 bis 1:3:3 beträgt, bei Temperaturen von 50 bis 140°C umsetzt, das Reaktionsprodukt sauer hydrolysiert und die anfallenden Säuren gegebenenfalls in an sich bekannter Weise in die wasserlöslichen Salze überführt.

3. Verfahren gemäß Anspruch 2, dadurch gekennzeichnet, daß man 2-Aminoisobuttersäure mit phosphoriger Säure und Phosphortrichlorid im Molverhältnis 1:1,5:1,5 umsetzt.

4. Verfahren gemäß Anspruch 2, dadurch gekennzeichnet, daß man Prolin mit phosphoriger Säure und Phosphortrichlorid im Molverhältnis von 1:2:2 umsetzt.

5. Verfahren gemäß Ansprüche 1 bis 4, dadurch gekennzeichnet, daß man die Umsetzung in Gegenwart cines inerten Lösungsmittels durchführt.

6. Verwendung von Hydroxydiphosphonsäuren der Formel I und deren wasserlösliche Salze als Komplexierungsmittel.

**Revendications**

1. Acides hydroxydiphosphoniques de formule générale

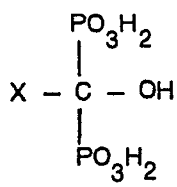

(I)

dans laquelle

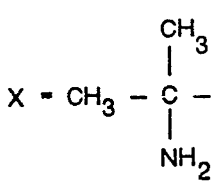

ou

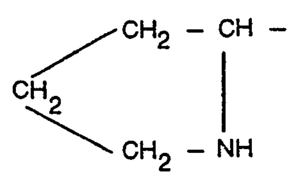

et leurs sels hydrosolubles.

2. Procédé de préparation des acides hydroxydiphosphoniques de formule I, caractérisé en ce que l'on fait réagir l'acide 2-aminoisobutyrique ou la proline avec l'acide phosphoreux et le trichlorure de phosphore à des proportions molaires de 1:1:1 à 1:3:3, à des températures de 50 à 140°C, on soumet le produit de réaction à hydrolyse acide et on convertit le cas échéant les acides obtenus en les sels hydrosolubles de manière connue en soi.

3. Procédé selon la revendication 2, caractérisé en ce que l'on fait réagir l'acide 2-aminoisobutyrique avec l'acide phosphoreux et le trichlorure de phosphore dans des proportions molaires de 1:1,5:1,5.

4. Procédé selon la revendication 2, caractérisé en ce que l'on fait réagir la proline avec l'acide phosphoreux et le trichlorure de phosphore dans des proportions molaires de 1:2:2.

5. Procédé selon les revendications 1 à 4, caractérisé en ce que l'on effectue la réaction en présence d'un solvant inerte.

6. Utilisation des acides hydroxydiphosphoniques de formule I et de leurs sels hydrosolubles en tant qu'agents complexants.

**Claims**

1. Hydroxydiphosphonic acids corresponding to the following general formula

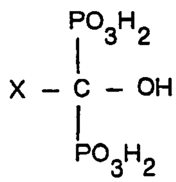

(I)

in which

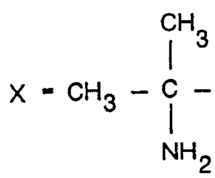

or

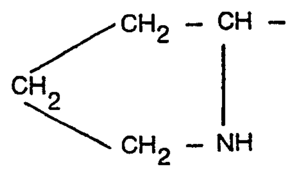

and their water-soluble salts.

2. A process for producing the hydroxydiphosphonic acids corresponding to formula I, characterised in that 2-aminoisobutyric acid or proline is reacted with phosphorous acid and phosphorus trichloride in a molar ratio of from 1:1:1 to 1:3:3 at temperatures in the range from 50 to 140°C, the reaction product is subjected to acid hydrolysis and the acids formed are optionally converted in known manner into the water-soluble salts.

3. A process as claimed in Claim 2, characterised in that 2-aminoisobutyric acid is reacted with phosphorus acid and phosphorus trichloride in a molar ratio of 1:1.5:1.5.

4. A process as claimed in Claim 2, characterised in that proline is reacted with phosphorus acid and phosphorus trichloride in a molar ratio of 1:2:2.

5. A process as claimed in Claims 1 to 4, characterised in that the reaction is carried out in the presence of an inert solvent.

6. The use of hydroxydiphosphonic acids corresponding to formula I and their water-soluble salts as complexing agents.